# EUROPEAN PATENT SPECIFICATION

(11) **EP 2 704 889 B1**
(45) Date of publication and mention of the grant of the patent: **25.05.2016**
(21) Application number: 12779955.9
(22) Date of filing: 18.04.2012
(51) Int. Cl.: B29C 33/12, A61M 5/34, B29C 45/14

(54) **MOLD ASSEMBLY AND METHOD FOR MANUFACTURING A SYRINGE CONTAINER**
GUSSFORM UND VERFAHREN ZUR HERSTELLUNG EINES SPRITZENBEHÄLTERS
ENSEMBLE MOULE ET PROCÉDÉ DE FABRICATION D'UN CONTENANT DE SERINGUE

(30) Priority: 03.05.2011 SE 1150384; 03.05.2011 US 201161481902 P
(43) Date of publication of application: 12.03.2014
(73) Proprietor: SHL Group AB, 131 28 Nacka Strand (SE)
(72) Inventor: DIETL, Thomas, 94350 Falkenfels (DE)
(86) International application number: PCT/SE2012/050414
(87) International publication number: WO 2012/150897

(56) References cited:
- EP-A1- 2 140 896
- EP-A1- 2 140 896
- FR-A- 1 149 526
- GB-A- 1 207 229
- JP-A- S 531 984
- JP-A- S60 158 968
- US-A- 3 330 004
- US-A- 3 336 654
- US-A- 3 336 654
- US-A- 4 314 960
- US-A- 5 510 065
- US-A1- 2010 270 702

## Description

### Field of the Invention

This invention generally relates to an injection molding method and mold assembly, and more particularly to a mold assembly and method for manufacturing a syringe container, more particular a polymeric syringe container.

### Background of the Invention

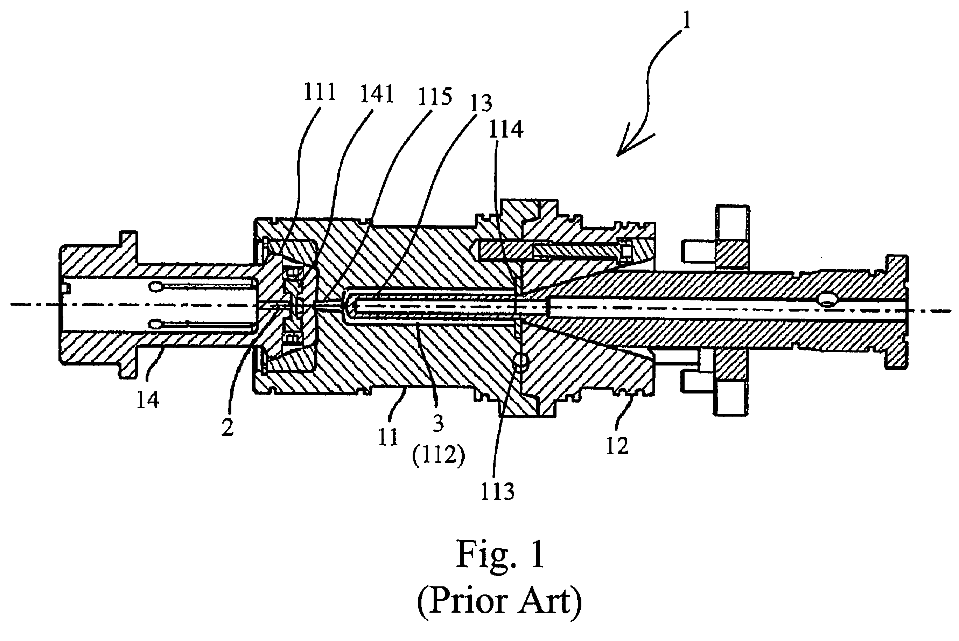

Fig. 1 is an insert mold 1 for molding a needle 2 to an injection barrel 3 disclosed in U.S. publication patent No. 2010/0270702 A1. The conventional insert mold 1 is provided with an A-side mold 11, a B-side mold 12, a core member 13 and a collect mechanism 14.

For the conventional molding process, one end of a needle 1 is placed into a tip of the core member 13 firstly. Next, the A-side mold 11 is inserted over the core member 13 to an extent that the A-side mold 11 contacts the B-side mold 12. Subsequently, the collect mechanism 14 is inserted in to a tapered receiving plenum 111 formed on left end of the A-side mold 11. When flexible arms 141 of the collect mechanism 14 hold the needle 2, molten polymeric material is injected into a mold space 112 defined between the A-side mold 11 and the B-side mold 12 through a gate 113 near broad end 114 of the mold space112. After the polymeric material in the mold space 112 is cooled, the collect mechanism 14 is removed out of the A-side mold 11. Then, the A-side mold 11 is separated from the B-side mold 12 so that the injection barrel 3 with the needle 2 can be taken out of the core member 13.

However, because the collect mechanism 14 and the A-side mold 11 are two separated parts in the conventional insert mold 1, a longitudinal axis of the collect mechanism 14 may not be in-line with a longitudinal axis of the mold space 112. Hence, a longitudinal axis of the needle 2 may not be in-line with a longitudinal axis of the injection barrel 3 after the molten polymeric material is injected.

Furthermore, a direction in which the flexible arms 141 move is perpendicular to a direction in which the collect mechanism 14 is inserted into or removed from the tapered receiving plenum 111. Therefore, when the collect mechanism 14 starts to move along its longitudinal axis, the arms 141 may not immediately separate from the needle 2, i.e. the arms 141 may still contact or hold the needle 2. In this way, the needle 2 will wear out contact surfaces of arms 141 which will affect clamping preciseness, or the arms 141 may bend or even damage the needle 2.

Moreover, on the ground that the gate 113 is located near the broad end 114 of the mold space 112, a converging end 115 of the mold space 112 may not be sufficiently filled with the molten polymeric material and thus be formed with defects of sink marks due to a drag of the molten polymeric material in the slender mold space 112. Even if the molten polymeric material can flow to the converging end 115, the pressure of polymeric material at the converging end 115 is still smaller than that at the broad end 114. Consequently, strength and dimension stability at the converging end 115 of the injection barrel 3 is low. What is worse is that a portion of the resultant injection barrel 3 corresponding to the converging end 115 cannot firmly hold the needle 2 as a result of the low pressure.

Besides, the core member 13 will be offset to incline to a longitudinal axis of a mold space 112 by the polymeric material injected into the mold space 112 through the single one gate 113.

In addition, the material of the contact surfaces of arms 141 is not so flexible to recover original shape after compression is released. The material has no sufficient durability against high pressure and temperature, neither. It is also difficult to precisely shape and thus fit the material of the contact surfaces to the arms.

Further solutions regarding die designs is disclosed e.g. in document EP 2 140 896. It discloses a method of producing a needle-equipped syringe barrel as well as a mold for such needle equipped syringe. The mold comprises an inner mold of a contour corresponding to an interior contour of an inner syringe barrel, which is provided with a concave portion for holding a rear end of a needle. Further an outer mold is arranged having a contour corresponding to exterior contours of an outer syringe barrel. A needle holder is fitted to elastically push the rear end of the needle to the concave portion. The mold is further arranged such that the rear end of the needle is pressed to the concave portion by pinning the proximal needle head against an elastic material or spring when the mold is closed.

Another document, FR 1 149 526 discloses a syringe needle assembly having an attachment part that is molded onto a distal end of the needle. It comprises a mold cavity in which a distal end of a needle is positioned. A core is introduced from the distal direction to form a conical attachment interior.

### Summary of the Invention

Accordingly, this invention relates to a mold assembly and method for manufacturing a syringe container which are substantially intended to obviate one or more of the problems due to the limitations and disadvantages encountered in the prior art.

One object of this invention is to provide a mold assembly and/or method which can make a longitudinal axis of a cannula in-line with a longitudinal axis of a syringe barrel before and after molten polymeric material is injected.

Another object of this invention is to provide a mold assembly and/or method which can avoid the clamping pieces from being worn out to affect clamping preciseness.

Yet another object of this invention is to provide a mold assembly and/or method which can avoid the cannula from being bent or damaged by the clamping pieces during a molding process.

A further object of this invention is to provide a mold assembly and/or method which can enhance the strength and dimension stability at a joint section of the syringe container.

Another object of this invention is to provide a mold assembly and/or method which can facilitate the joint section to firmly hold the cannula of the syringe container.

Yet another object of this invention is to provide a mold assembly and/or method which can maintain a longitudinal axis of a core in-line with a longitudinal axis of a mold cavity.

Another object of this invention is to provide material for clamping pieces of a mold assembly and/or method which has properties as follows: flexibility for recovering original shape after compression is released, durability against high pressure and temperature, possibility to be precisely shaped, low thermal expansion, high elasticity for preventing from damaging the surface of the cannula, and nonstick property.

These objects are achieved by mold assembly as defined by claim 1 and a method as defined by claim 11. The dependent claims define preferred or advantageous embodiments of the mold assembly and method.

Additional features and advantages of the invention will be set forth in the description which follows, and in portion will be apparent from the description, or may be learned by practice of the invention. The objectives and advantages of the invention will be realized and attained by the structure as particularly set forth in the written description and claims as well as illustrated in the appended drawings.

To achieve these and other advantages and according to the purpose of this invention, as embodied and broadly described, a mold assembly for manufacturing a syringe container includes: a first set; a second set movable to abut against the first set to define a mold cavity therebetween; a core located in the mold cavity; and a first clamping piece and a second clamping piece for holding the cannula; characterized in that the first and second clamping pieces are respectively secured to the first and second sets.

Another aspect of this invention directs to a method for manufacturing a syringe container with a mold assembly. The method includes: placing one end of a cannula into a port of a core; moving a first and second sets; injecting polymeric material into a mold cavity; separate the first and second sets from the core; and removing the syringe container out of the core; characterized in that: a first and second clamping pieces perform a function of holding the cannula the moment that the first and second sets are moved to abut against each other.

These preferred embodiments can make a longitudinal axis of a cannula in-line with a longitudinal axis of a syringe barrel, avoid the clamping pieces from being worn out to affect clamping preciseness, and avoid the cannula from being damaged by the clamping pieces.

Moreover, the mold cavity includes an engagement cavity corresponding to a joint section and a bore cavity corresponding to an annular section along a longitudinal axis of the core. The first set is formed with a first gate opening into the engagement cavity. The preferred embodiment can enhance the strength and dimension stability at the joint section of the syringe container, and facilitate the joint section to firmly hold the cannula of the syringe container.

Furthermore, the second set is formed with a second gate opening into the engagement cavity. The first gate and the second gate are symmetrical about the longitudinal axis of the core

The polymeric material is injected into the mold cavity through a portion of the mold cavity which accommodates one end of the cannula and/or one end of the core formed with the port.

The polymeric material is injected into the mold cavity through the first gate and the second gate which open into the portion of the mold cavity and are symmetrical about a longitudinal axis of the core.

The first gate and the second gate are respectively formed in the first set and the second set.

A portion of the engagement cavity and a portion of the bore cavity are formed in the first set. Another portion of the engagement cavity and another portion of the bore cavity are formed in the second set.

These preferred embodiments can maintain the longitudinal axis of the core in-line with the longitudinal axis of the mold cavity because the molten polymeric material flow into the mold cavity from two sides of the core and the pressures in the two sides of the core are the same.

It is another preferred feature that the first clamping piece and the second clamping piece are made of a plastic material. This specific material for the clamping pieces has properties as follows: flexibility for recovering original shape after compression is released, durability against high pressure and temperature, possibility to be precisely shaped, low thermal expansion, high elasticity for preventing from damaging the surface of the cannula, and nonstick property.

It is preferred that the first set further includes a first main block and a first sub-block. The second set further includes a second main block and a second sub-block. The bore cavity is formed between the first main block and the second main block. The engagement cavity is formed between the first sub-block and the second sub-block.

The first set includes two of the first sub-blocks. The second set includes two of the second sub-blocks. The first gate is formed between the first sub-blocks. The second gate is formed between the second sub-blocks.

The first clamping piece is formed with a first slot in which a portion of the cannula abuts against the first clamping piece.

The first set is formed with a first trough communicating the first slot with the mold cavity.

The core is formed with a port at one end thereof. One end of the cannula is arranged in the port and another end of the cannula is outside the mold assembly.

It is to be understood that both the foregoing general description and the following detailed description are exemplary and explanatory and are intended to provide a further non-limiting explanation of the invention as claimed.

### Brief Description of the Drawings

The accompanying drawings, which are included to provide a further understanding of the invention and constitute a portion of the specification, illustrate embodiments of the invention and together with the description serve to explain the principle of the invention. In the drawings:
Fig. 1 is an assembled sectional view illustrating a conventional insert mold;
Fig. 2 is an assembled sectional view illustrating a syringe container manufactured by a mold assembly and method according to this invention;
Fig. 3 is an assembled perspective view of the mold assembly according to this invention illustrating an open position;
Fig. 4 is an exploded perspective view illustrating a first set of the mold assembly in the Fig. 3; and
Fig. 5 is an assembled sectional view of the mold assembly taken along a horizontal plane passing a longitudinal axis of a core according to this invention and illustrating a close position.

### Detailed Description of the Invention

### Syringe Container

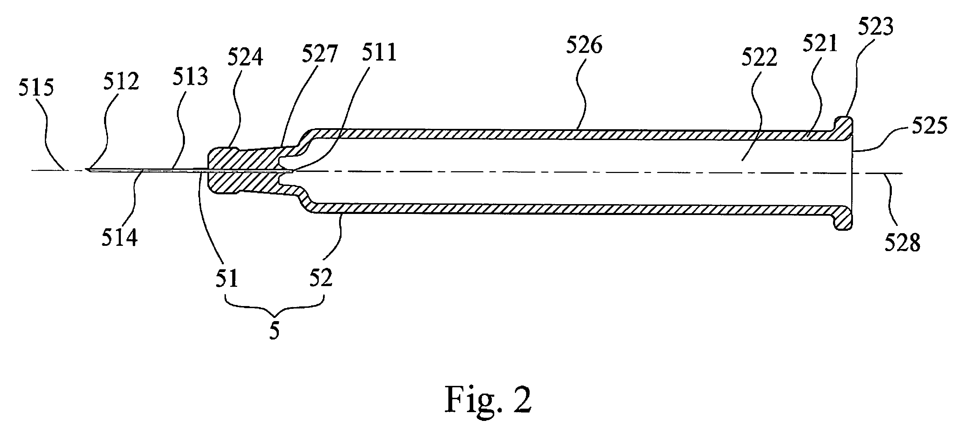

As shown in Fig. 2, a syringe container 5 of this invention may include a hollow cannula 51 and a substantially cylindrical syringe barrel 52. The cannula 51 is made of, for example, metal and may have a blunt end 511, a sharp end 512, a middle portion 513 between the blunt end 51 land the sharp end 512, and a passage 514 through the cannula 51 from the blunt end 511 to the sharp end 512.

The syringe barrel 52 may include a wall 521 formed with, for example, polymeric material and a chamber 522 defined by the wall 521. The profile of the syringe barrel 52 may show a wide end 523, a narrow end 524, an opening 525 defined by the wall 521 at the wide end 523, an annular section 526 near the wide end 523, and a joint section 527 extending to the narrow end 524 from another end of the annular section 526 opposite to the opening 525. The joint section 527 may have a plurality of steps with different diameters respectively.

The syringe barrel 52 may be molded around a portion of the cannula 51 such that the blunt end 511 of the cannula 51 is located in the chamber 522 of the syringe barrel 52, a portion near the blunt end 511 of the cannula 51 is firmly secured by the joint section 527 of the syringe barrel 52, the sharp end 512 is situated outside the syringe barrel 52, and the passage 514 of the cannula is communicated with the chamber 522 of the syringe barrel 52. A longitudinal axis 515 of the cannula 51 is in-line or in alignment with a longitudinal axis 528 of the syringe barrel 52.

### Mold Assembly

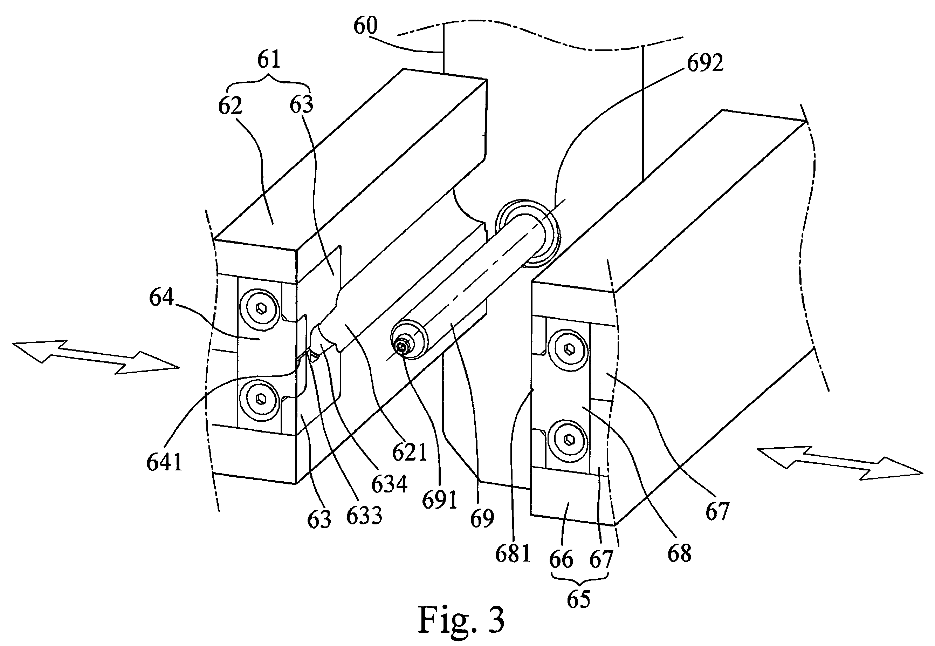

As shown in Fig. 3, a mold assembly 6 for manufacturing the syringe container 5 of this invention may include a base 60, a first set 61 movable relative to the base 60, a first clamping piece 64 firmly secured to the first set 61, a second set 65 movable relative to the base 60, a second clamping piece 68 firmly secured to the second set 65, and a pin-shaped core 69 firmly secured to the base 60 and disposed between the first set 61 and the second set 65.

Outer profile of the core 69 corresponds to inner profile of the syringe barrel 52. The core 69 is made of, for example, metal and may be formed with a taped receiving port 691 at one end thereof opposite to the base 60.

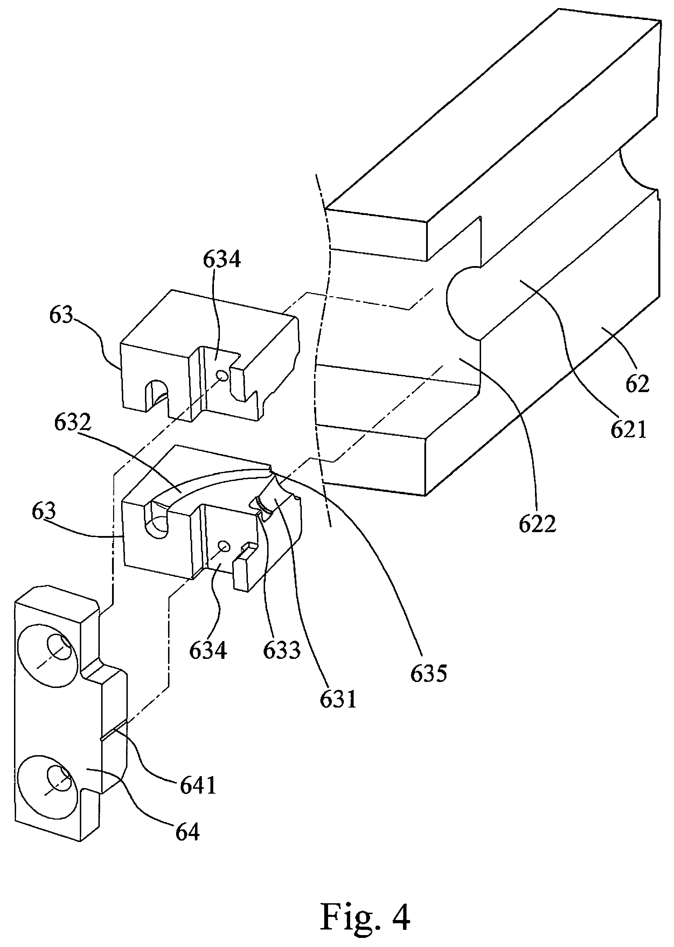

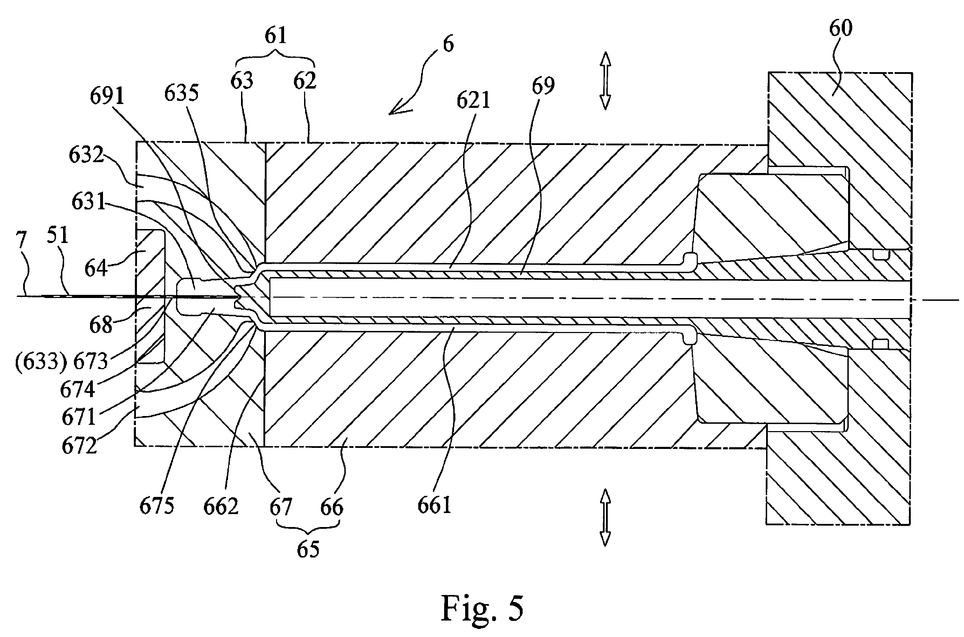

As shown in Figs. 3, 4 and 5, the first set 61 may further include a first main block 62 and two first sub-blocks 63 firmly secured to the first main block 62. The first main block 62 is made of, for example, metal. The first main block 62 may be formed with a first bore cavity 621 whose longitudinal direction is parallel to a longitudinal axis 692 of the core 69 and a first recess 622 which opens into the first bore cavity 621 near the port 691 of the core 69. Inner profile of the first bore cavity 621 corresponds to half outer profile of the annular section 526 of the syringe barrel 52.

The first sub-blocks 63 are made of, for example, metal. Each of the first sub-blocks 63 may be formed with a first engagement cavity half 631, a first runner half 632 communicated with the first engagement cavity half 631 near the first bore cavity 621, a first trough half 633 communicated with the first engagement cavity half 631 at another end thereof, and a first receptacle half 634 communicated with the first trough half 633 at another end thereof. Inner profile of the first engagement cavity half 631 corresponds to one-fourth outer profile of the joint section 527 of the syringe barrel 52 sectioned along the longitudinal direction. The structures of the two first sub-blocks 63 may be in a relation of plane symmetry. When the two first sub-blocks 63 are amounted into the first recess 622 and firmly secured to the first main block 62, the two first runner halves 632 constitute a runner 632 between the two first sub-blocks 63. The two first engagement cavity halves 631 also constitute a first engagement cavity 631which opens into the first bore cavity 621. The location where the first runner 632 connects the first engagement cavity 631 forms a first gate 635 to communicate the first runner 632 with the first engagement cavity 631. The two first trough halves 633 and two first receptacle halves 634 respectively constitute a first trough 633 and first receptacle 634, too. The first engagement cavity 631 and the first bore cavity 621 constitute a first mold cavity. Alternatively, two of the first sub-blocks 63 may be integrated into a one-piece member. Two of the second sub-blocks 67 may be integrated into a one-piece member as well.

The first clamping piece 64 may be formed with a first slot 641. The first clamping piece 64 is received in the first receptacle 634 and firmly secured to the two first sub-blocks 63 such that the first slot 641 is aligned with the first trough 633. In other words, the first clamping piece 64 may be built in the first sub-block 63 or the first set 61. The first clamping piece 64 is made ofa plastic material, wherein this specific material for the clamping pieces has properties as follows: flexibility for recovering original shape after compression is released, durability against high pressure and temperature, possibility to be precisely shaped, low thermal expansion, high elasticity for preventing from damaging the surface of the cannula, and nonstick property.

The first and second sets 61, 65 may be symmetrical with respect to a virtual parting plane 7 therebetween, on which the longitudinal axis 692 of the core 69 may be situated. Therefore, the second set 65 may include a second main block 66 and two second sub-blocks 67. The second main block 66 may be formed with a second bore cavity 661 and a second recess 662. Each of the second sub-block 67 may be formed with a second engagement cavity 671, second runner 672, second trough 673, second receptacle 674 and second gate 675. The second clamping piece 68 may be also formed with a second slot 681.

The first and second gates 635, 675 may communicate respectively with the first and second engagement cavities 631, 671 at different steps thereof. However, the first gate 635 and the second gate 675 are preferably symmetrical about the longitudinal axis 692 of the core 69.

### Process

The method for manufacturing the syringe container 5 of this invention may include the following steps. First, an End of Arm in an automation system (not shown) transports the cannula 51 and places the blunt end 511 into the port 691 of the core 69 such that the longitudinal axis 515 of cannula 51 is substantially in-line with the longitudinal axis 692 of core 69.

Next, an injection molding machine (not shown) actuates the first main block 62 and the second main block 66 to move toward the core 69. As mentioned above, the first and second sub-blocks 63, 67 are respectively secured to the first and second main blocks 62, 66, and the first and second clamping pieces 64, 68 are respectively secured to the first and second sub-blocks 63, 67. Therefore, when the first main block 62 abuts against the second main block 66, the first sub-block 63 also abuts against the second sub-block 67 to define a close position of the mold assembly 6 and the virtual parting plane 7 between the first and second sets 61, 65. In the close position, the first and second bore cavities 621, 661 constitute a bore cavity, the first and second engagement cavities 631, 671 constitute an engagement cavity, and the first and second troughs 633, 673 constitute a trough. Moreover, the engagement cavity and bore cavity constitutes a mold cavity, in which the core 69 is situated.

In the close position, the first clamping piece 64 may or may not abut against the second clamping piece 68 but the first and second clamping pieces 64, 68 clamp the middle section 513 of the cannula 51 in the first and second slots 641, 681 with a proper force such that the longitudinal axis 515 of the cannula 51 is in-line with the longitudinal axis of the bore cavity and engagement cavity. The blunt end 511 is situated in the engagement cavity. The sharp end 512 protrudes outside the first and second clamping pieces 64, 68. The inner diameters of the first and second troughs 633, 673 may be a little bit larger than those of the first and second slots 641, 681 so that the first and second sub-blocks 63, 67 hardly clamp the cannula 51.

After the first and second clamping pieces 64, 68 hold the cannula 51, the End of Arm releases the cannula 51 such that the longitudinal axes 515, 692 of the cannula 51 and core 69 and the longitidunal axis of the mold cavity are in-line. Subsequently, molten polymeric material is injected through the first and second runners 632, 672, the first and second gates 635, 675 and then into the empty engagement cavity and bore cavity. When the polymeric material in the mold cavity is cooled to a substantially solidified state, the first and second sets 61, 65 are separated along the parting plane 7 and define an open position of the mold assembly 6. Then, the resultant syringe container 5 is taken out of the core 69.

This invention has been disclosed in terms of specific embodiments. It will be apparent that many modifications can be made to the disclosed structures and steps without departing from the invention. Therefore, it is the intent of the appended claims to cover all such variations and modifications that are within the scope of this invention.

## Claims

1. A mold assembly (6) for manufacturing a syringe container (5) which is provided with a cannula (51) and a syringe barrel (52), the syringe barrel (52) being formed with an annular section (526) and a joint section (527) which holds the cannula (51), the mold assembly (6) comprising:
a first set (61);
a second set (65) movable to abut against the first set (61) to define a mold cavity (621,631, 661,671) therebetween;
a core (69) located in the mold cavity (621,631, 661,671); and
a first clamping piece (64) and a second clamping piece (68) for holding the cannula (51);
**characterized in that**
the first and second clamping pieces (64, 68) are respectively secured to the first and second sets (61, 65).

2. The mold assembly for manufacturing a syringe container according to the claim 1, **characterized in that** the mold cavity (621,631, 661,671) includes an engagement cavity (631, 671) corresponding to the joint section (527) and a bore cavity (621, 661) corresponding to the annular section (526) along a longitudinal axis (692) of the core (69); the first set (61) is formed with a first gate (635) opening into the engagement cavity (631, 671).

3. The mold assembly for manufacturing a syringe container according to the claim 2, **characterized in that** the second set (65) is formed with a second gate (675) opening into the engagement cavity (631, 671); the first gate (635) and the second gate (675) are symmetrical about the longitudinal axis (692) of the core (69).

4. The mold assembly for manufacturing a syringe container according to the claim 3, **characterized in that** a portion of the engagement cavity (631, 671) and a portion of the bore cavity (621, 661) are formed in the first set (61); and another portion of the engagement cavity (631, 671) and another portion of the bore cavity (621, 661) are formed in the second set (65).

5. The mold assembly for manufacturing a syringe container according to the claim 4, **characterized in that** the first set (61) further includes a first main block (62) and a first sub-block (63); the second set (65) further includes a second main block (66) and a second sub-block (67); the bore cavity (621, 661) is formed between the first main block (62) and the second main block (66); the engagement cavity (631, 671) is formed between the first sub-block (63) and the second sub-block (67).

6. The mold assembly for manufacturing a syringe container according to the claim 5, **characterized in that** the first set (61) includes two of the first sub-blocks (63); the second set (65) includes two of the second sub-blocks (67); the first gate (635) is formed between the first sub-blocks (63); the second gate (675) is formed between the second sub-blocks (67).

7. The mold assembly for manufacturing a syringe container according to any one of the claims 1 to 6, **characterized in that** the first clamping piece (64) and the second clamping piece (68) are made of a plastic material.

8. The mold assembly for manufacturing a syringe container according to the claim 7, **characterized in that** the first clamping piece (64) is formed with a first slot (641) in which a portion of the cannula (51) abuts against the first clamping piece (64).

9. The mold assembly for manufacturing a syringe container according to the claim 8, **characterized in that** the first set (61) is formed with a first trough (633) communicating the first slot (641) with the mold cavity (621,631, 661,671).

10. The mold assembly for manufacturing a syringe container according to the claim 9, **characterized in that** the core (69) is formed with a port (691) at one end thereof; one end of the cannula (51) is arranged in the port (691) and another end of the cannula (51) is outside the mold assembly (6).

11. A method for manufacturing a syringe container (5) with a mold assembly (6),
the syringe container (5) being provided with a cannula (51) and a syringe barrel (52) holding the cannula (51);
the mold assembly (6) including: a first set (61); a first clamping piece (64); a second set (65) which together with the first set (61) define a mold cavity (621,631, 661,671) corresponding to an outer profile of the syringe barrel (52); a second clamping piece (68) facing the first clamping piece (64); and a core (69) between the first set (61) and the second set (65);
the method comprising:
placing one end of the cannula (51) into a port (691) of the core (69);
moving the first and second sets (61, 65);
injecting polymeric material into the mold cavity (621,631, 661,671);
separate the first and second sets (61, 65) from the core (69); and
removing the syringe container (5) out of the core (69);
**characterized in that**:
the first and second clamping pieces (64, 68) perform a function of holding the cannula (51) the moment that the first and second sets (61, 65) are moved to abut against each other.

12. The method for manufacturing asyringe container with a mold assembly according to the claim 11, **characterized in that** the first and second clamping pieces (64, 68) are respectively secured to the first and second sets (61, 65).

13. The method for manufacturing a syringe container with a mold assembly according to the claim 12, **characterized in that** the polymeric material is injected into the mold cavity (621,631, 661,671) through a portion of the mold cavity which accommodates the one end of the cannula (51) and/or one end of the core (69) formed with the port (691).

14. The method for manufacturing a syringe container with a mold assembly according to the claim 13, **characterized in that** the polymeric material is injected into the mold cavity (621,631, 661,671) through a first gate (635) and a second gate (675) which open into the portion of the mold cavity (621,631, 661,671) and are symmetrical about a longitudinal axis (692) of the core (69).

15. The method for manufacturing a syringe container with a mold assembly according to the claim 14, **characterized in that** the first gate (635) and the second gate (675) are respectively formed in the first set (61) and the second set (65).

16. The method for manufacturing a syringe container with a mold assembly according to the claim 15, **characterized in that** the first set (61) further includes a first main block (62) and a first sub-block (63); the second set (65) further includes a second main block (66) and a second sub-block (67); the portion of the mold cavity (621,631, 661,671) is formed between the first sub-block (63) and the second sub-block (67); another portion of the mold cavity (621,631, 661,671) which does not receiving the cannula (51) is formed between the first main block (62) and the second main block (66).

17. The method for manufacturing a syringe container with a mold assembly according to the claim 16, **characterized in that** the first set (61) includes two of the first sub-blocks (63); the second set (65) includes two of the second sub-blocks (67); the first gate (635) is formed between the first sub-blocks (63); the second gate (675) is formed between the second sub-blocks (67).

18. The method for manufacturing a syringe container with a mold assembly according to any one of the claims 11 to 17, **characterized in that** the first clamping piece (64) and the second clamping piece (68) are made of a plastic material.

19. The method for manufacturing a syringe container with a mold assembly according to the claim 18, **characterized in that** the first clamping piece (64) is formed with a first slot (641) in which a portion of the cannula (51) abuts against the first clamping piece (64).

20. The method for manufacturing a syringe container with a mold assembly according to the claim 19, **characterized in that** the first set (61) is formed with a first trough (633) communicating the first slot (641) with the mold cavity (621,631, 661,671).

21. The method for manufacturing a syringe container with a mold assembly according to the claim 20, **characterized in that** another end of the cannula (51) is outside the mold assembly (6) and a middle portion (513) of the cannula (51) is received in the first trough (633).

## Patentansprüche

1. Gussform (6) zur Herstellung eines Spritzenbehälters (5), der mit einer Hohlnadel (51) und einem Spritzenzylinder (52) versehen ist, wobei der Spritzenzylinder (52) mit einem ringförmigen Abschnitt (526) und einem Verbindungsabschnitt (527), der die Hohlnadel (51) hält, versehen ist, wobei die Gussform (6) Folgendes umfasst:
einen ersten Satz (61);
einen zweiten Satz (65), der so bewegbar ist, dass er an den ersten Satz (61) anstößt, damit sie einen Formhohlraum (621, 631, 661, 671) dazwischen festlegen;
einen Kern (69), der in dem Formhohlraum (621, 631, 661, 671) befindlich ist; und
ein erstes Klemmteil (64) und ein zweites Klemmteil (68) zum Halten der Hohlnadel (51);
**dadurch gekennzeichnet, dass**
das erste und zweite Klemmteil (64, 68) an dem ersten beziehungsweise zweiten Satz (61, 65) befestigt sind.

2. Gussform zur Herstellung eines Spritzenbehälters nach Anspruch 1, **dadurch gekennzeichnet, dass** der Formhohlraum (621, 631, 661, 671) einen Eingriffhohlraum (631, 671), der dem Verbindungsabschnitt (527) entspricht, sowie einen Bohrungshohlraum (621, 661), der dem ringförmigen Abschnitt (526) entspricht, entlang einer Längsachse (692) des Kerns (69) aufweist; der erste Satz (61) mit einer ersten Anbindung (635) versehen ist, die in den Eingriffhohlraum (631, 671) mündet.

3. Gussform zur Herstellung eines Spritzenbehälters nach Anspruch 2, **dadurch gekennzeichnet, dass** der zweite Satz (65) mit einer zweiten Anbindung (675) versehen ist, die in den Eingriffhohlraum (631, 671) mündet; die erste Anbindung (635) und die zweite Anbindung (675) um die Längsachse (692) des Kerns (69) symmetrisch sind.

4. Gussform zur Herstellung eines Spritzenbehälters nach Anspruch 3, **dadurch gekennzeichnet, dass** ein Abschnitt des Eingriffhohlraums (631, 671) und ein Abschnitt des Bohrungshohlraums (621, 661) in dem ersten Satz (61) ausgebildet sind; und ein weiterer Abschnitt des Eingriffhohlraums (631, 671) und ein weiterer Abschnitt des Bohrungshohlraums (621, 661) in dem zweiten Satz (65) ausgebildet sind.

5. Gussform zur Herstellung eines Spritzenbehälters nach Anspruch 4, **dadurch gekennzeichnet, dass** der erste Satz (61) ferner einen ersten Hauptblock (62) und einen ersten Teilblock (63) aufweist; der zweite Satz (65) ferner einen zweiten Hauptblock (66) und einen zweiten Teilblock (67) aufweist; der Bohrungshohlraum (621, 661) zwischen dem ersten Hauptblock (62) und dem zweiten Hauptblock (66) ausgebildet ist; der Eingriffhohlraum (631, 671) zwischen dem ersten Teilblock (63) und dem zweiten Teilblock (67) ausgebildet ist.

6. Gussform zur Herstellung eines Spritzenbehälters nach Anspruch 5, **dadurch gekennzeichnet, dass** der erste Satz (61) zwei der ersten Teilblöcke (63) aufweist; der zweite Satz (65) zwei der zweiten Teilblöcke (67) aufweist; die erste Anbindung (635) zwischen den ersten Teilblöcken (63) ausgebildet ist; die zweite Anbindung (675) zwischen den zweiten Teilblöcken (67) ausgebildet ist.

7. Gussform zur Herstellung eines Spritzenbehälters nach einem der Ansprüche 1 bis 6, **dadurch gekennzeichnet, dass** das erste Klemmteil (64) und das zweite Klemmteil (68) aus einem Kunststoff gefertigt sind.

8. Gussform zur Herstellung eines Spritzenbehälters nach Anspruch 7, **dadurch gekennzeichnet, dass** das erste Klemmteil (64) mit einem ersten Schlitz (641) versehen ist, in dem ein Abschnitt der Hohlnadel (51) an dem ersten Klemmteil (64) anliegt.

9. Gussform zur Herstellung eines Spritzenbehälters nach Anspruch 8, **dadurch gekennzeichnet, dass** der erste Satz (61) mit einem ersten Kanal (633) versehen ist, der den ersten Schlitz (641) mit dem Formhohlraum (621, 631, 661, 671) in Verbindung bringt.

10. Gussform zur Herstellung eines Spritzenbehälters nach Anspruch 9, **dadurch gekennzeichnet, dass** der Kern (69) an einem Ende davon mit einer Öffnung (691) versehen ist; ein Ende der Hohlnadel (51) in der Öffnung (691) angeordnet ist und sich ein weiteres Ende der Hohlnadel (51) außerhalb der Gussform (6) befindet.

11. Verfahren zur Herstellung eines Spritzenbehälters (5) mit einer Gussform (6),
wobei der Spritzenbehälter (5) mit einer Hohlnadel (51) und einem Spritzenzylinder (52), der die Hohlnadel (51) hält, versehen ist;
wobei die Gussform (6) Folgendes aufweist: einen ersten Satz (61); ein erstes Klemmteil (64); einen zweiten Satz (65), der zusammen mit dem ersten Satz (61) einen Formhohlraum (621, 631, 661, 671) festlegt, der einem Außenprofil des Spritzenzylinders (52) entspricht; ein zweites Klemmteil (68) gegenüber dem ersten Klemmteil (64); und einen Kern (69) zwischen dem ersten Satz (61) und dem zweiten Satz (65);
wobei das Verfahren Folgendes umfasst:
Platzieren eines Endes der Hohlnadel (51) in einer Öffnung (691) des Kerns (69);
Bewegen des ersten und zweiten Satzes (61, 65);
Einspritzen von Polymermaterial in den Formhohlraum (621, 631, 661, 671);
Trennen des ersten und zweiten Satzes (61, 65) vom Kern (69); und
Entnehmen des Spritzenbehälters (5) aus dem Kern (69);
**dadurch gekennzeichnet, dass**:
das erste und zweite Klemmteil (64, 68) in dem Moment, wenn der erste und zweite Satz (61, 65) so bewegt werden, dass sie aneinander anstoßen, eine Funktion des Haltens der Hohlnadel (51) erfüllen.

12. Verfahren zur Herstellung eines Spritzenbehälters mit einer Gussform nach Anspruch 11, **dadurch gekennzeichnet, dass** das erste und zweite Klemmteil (64, 68) an dem ersten beziehungsweise zweiten Satz (61, 65) befestigt sind.

13. Verfahren zur Herstellung eines Spritzenbehälters mit einer Gussform nach Anspruch 12, **dadurch gekennzeichnet, dass** das Polymermaterial durch einen Abschnitt des Formhohlraums, der das eine Ende der Hohlnadel (51) und/oder ein Ende des Kerns (69) aufnimmt, das mit der Öffnung (691) versehen ist, in den Formhohlraum (621, 631, 661, 671) gespritzt wird.

14. Verfahren zur Herstellung eines Spritzenbehälters mit einer Gussform nach Anspruch 13, **dadurch gekennzeichnet, dass** das Polymermaterial durch eine erste Anbindung (635) und eine zweite Anbindung (675), die in den Abschnitt des Formhohlraums (621, 631, 661, 671) münden und um eine Längsachse (692) des Kerns (69) herum symmetrisch sind, in den Formhohlraum (621, 631, 661, 671) gespritzt wird.

15. Verfahren zur Herstellung eines Spritzenbehälters mit einer Gussform nach Anspruch 14, **dadurch gekennzeichnet, dass** die erste Anbindung (635) und die zweite Anbindung (675) in dem ersten Satz (61) beziehungsweise dem zweiten Satz (65) ausgebildet sind.

16. Verfahren zur Herstellung eines Spritzenbehälters mit einer Gussform nach Anspruch 15, **dadurch gekennzeichnet, dass** der erste Satz (61) ferner einen ersten Hauptblock (62) und einen ersten Teilblock (63) aufweist; der zweite Satz (65) ferner einen zweiten Hauptblock (66) und einen zweiten Teilblock (67) aufweist; der Abschnitt des Formhohlraums (621, 631, 661, 671) zwischen dem ersten Teilblock (63) und dem zweiten Teilblock (67) ausgebildet ist; ein weiterer Abschnitt des Formhohlraums (621, 631, 661, 671), der die Hohlnadel (51) nicht aufnimmt, zwischen dem ersten Hauptblock (62) und dem zweiten Hauptblock (66) ausgebildet ist.

17. Verfahren zur Herstellung eines Spritzenbehälters mit einer Gussform nach Anspruch 16, **dadurch gekennzeichnet, dass** der erste Satz (61) zwei der ersten Teilblöcke (63) aufweist; der zweite Satz (65) zwei der zweiten Teilblöcke (67) aufweist; die erste Anbindung (635) zwischen den ersten Teilblöcken (63) ausgebildet ist; die zweite Anbindung (675) zwischen den zweiten Teilblöcken (67) ausgebildet ist.

18. Verfahren zur Herstellung eines Spritzenbehälters mit einer Gussform nach einem der Ansprüche 11 bis 17, **dadurch gekennzeichnet, dass** das erste Klemmteil (64) und das zweite Klemmteil (68) aus einem Kunststoff gefertigt sind.

19. Verfahren zur Herstellung eines Spritzenbehälters mit einer Gussform nach Anspruch 18, **dadurch gekennzeichnet, dass** das erste Klemmteil (64) mit einem ersten Schlitz (641) versehen ist, in dem ein Abschnitt der Hohlnadel (51) an dem ersten Klemmteil (64) anliegt.

20. Verfahren zur Herstellung eines Spritzenbehälters mit einer Gussform nach Anspruch 19, **dadurch gekennzeichnet, dass** der erste Satz (61) mit einem ersten Kanal (633) versehen ist, der den ersten Schlitz (641) mit dem Formhohlraum (621, 631, 661, 671) in Verbindung bringt.

21. Verfahren zur Herstellung eines Spritzenbehälters mit einer Gussform nach Anspruch 20, **dadurch gekennzeichnet, dass** sich ein weiteres Ende der Hohlnadel (51) außerhalb der Gussform (6) befindet und ein mittlerer Abschnitt (513) der Hohlnadel (51) in dem ersten Kanal (633) aufgenommen ist.

## Revendications

1. Ensemble moule (6) destiné à la fabrication d'un contenant de seringue (5) qui est doté d'une canule (51) et d'un cylindre de seringue (52), le cylindre de seringue (52) étant formé avec une section annulaire (526) et une section de liaison (527) qui maintient la canule (51), l'ensemble moule (6) comprenant :
un premier ensemble (61) ;
un second ensemble (65) mobile pour venir en butée contre le premier ensemble (61) afin de définir une cavité de moule (621, 631, 661, 671) entre eux ;
un noyau (69) situé dans la cavité de moule (621, 631, 661, 671) ; et
une première pièce de serrage (64) et une seconde pièce de serrage (68) pour maintenir la canule (51) ;
**caractérisé en ce que**
les première et seconde pièces de serrage (64, 68) sont fixées respectivement aux premier et second ensembles (61, 65).

2. Ensemble moule destiné à la fabrication d'un contenant de seringue selon la revendication 1, **caractérisé en ce que** la cavité de moule (621, 631, 661, 671) comporte une cavité d'engagement (631, 671) correspondant à la section de liaison (527) et une cavité d'alésage (621, 661) correspondant à la section annulaire (526) le long d'un axe longitudinal (692) du noyau (69) ; le premier ensemble (61) est formé avec un premier point d'injection (635) s'ouvrant dans la cavité d'engagement (631, 671).

3. Ensemble moule destiné à la fabrication d'un contenant de seringue selon la revendication 2, **caractérisé en ce que** le second ensemble (65) est formé avec un second point d'injection (675) s'ouvrant dans la cavité d'engagement (631, 671) ; le premier point d'injection (635) et le second point d'injection (675) sont symétriques autour de l'axe longitudinal (692) du noyau (69).

4. Ensemble moule destiné à la fabrication d'un contenant de seringue selon la revendication 3, **caractérisé en ce qu'**une partie de la cavité d'engagement (631, 671) et une partie de la cavité d'alésage (621, 661) sont formées dans le premier ensemble (61) ; et une autre partie de la cavité d'engagement (631, 671) et une autre partie de la cavité d'alésage (621, 661) sont formées dans le second ensemble (65).

5. Ensemble moule destiné à la fabrication d'un contenant de seringue selon la revendication 4, **caractérisé en ce que** le premier ensemble (61) comporte en outre un premier bloc principal (62) et un premier sous-bloc (63) ; le second ensemble (65) comporte en outre un second bloc principal (66) et un second sous-bloc (67) ; la cavité d'alésage (621, 661) est formée entre le premier bloc principal (62) et le second bloc principal (66) ; la cavité d'engagement (631, 671) est formée entre le premier sous-bloc (63) et le second sous-bloc (67).

6. Ensemble moule destiné à la fabrication d'un contenant de seringue selon la revendication 5, **caractérisé en ce que** le premier ensemble (61) comporte deux des premiers sous-blocs (63) ; le second ensemble (65) comporte deux des seconds sous-blocs (67) ; le premier point d'injection (635) est formé entre les premiers sous-blocs (63) ; le second point d'injection (675) est formé entre les seconds sous-blocs (67).

7. Ensemble moule destiné à la fabrication d'un contenant de seringue selon l'une quelconque des revendications 1 à 6, **caractérisé en ce que** la première pièce de serrage (64) et la seconde pièce de serrage (68) sont fabriquées en une matière plastique.

8. Ensemble moule destiné à la fabrication d'un contenant de seringue selon la revendication 7, **caractérisé en ce que** la première pièce de serrage (64) est formée avec une première fente (641) dans laquelle une partie de la canule (51) vient en butée contre la première pièce de serrage (64).

9. Ensemble moule destiné à la fabrication d'un contenant de seringue selon la revendication 8, **caractérisé en ce que** le premier ensemble (61) est formé avec un premier conduit (633) qui fait communiquer la première fente (641) avec la cavité de moule (621, 631, 661, 671).

10. Ensemble moule destiné à la fabrication d'un contenant de seringue selon la revendication 9, **caractérisé en ce que** le noyau (69) est formé avec un orifice (691) sur une de ses extrémités ; une extrémité de la canule (51) est agencée dans l'orifice (691) et une autre extrémité de la canule (51) est à l'extérieur de l'ensemble moule (6).

11. Procédé de fabrication d'un contenant de seringue (5) avec un ensemble moule (6),
le contenant de seringue (5) étant doté d'une canule (51) et d'un cylindre de seringue (52) maintenant la canule (51) ;
l'ensemble moule (6) comportant : un premier ensemble (61) ; une première pièce de serrage (64) ; un second ensemble (65) qui, avec le premier ensemble (61), définit une cavité de moule (621, 631, 661, 671) correspondant à un profil extérieur du cylindre de seringue (52) ; une seconde pièce de serrage (68) faisant face à la première pièce de serrage (64) ; et un noyau (69) entre le premier ensemble (61) et le second ensemble (65) ;
le procédé comprenant :
placer une extrémité de la canule (51) dans un orifice (691) du noyau (69);
déplacer les premier et second ensembles (61, 65) ;
injecter un matériau polymère dans la cavité de moule (621, 631, 661, 671);
séparer les premier et second ensembles (61, 65) du noyau (69) ; et
retirer le contenant de seringue (5) du noyau (69) ;
**caractérisé en ce que** :
les première et seconde pièces de serrage (64, 68) effectuent une fonction de maintien de la canule (51) au moment où les premier et second ensembles (61, 65) sont déplacés pour venir en butée l'un contre l'autre.

12. Procédé de fabrication d'un contenant de seringue avec un ensemble moule selon la revendication 11, **caractérisé en ce que** les première et seconde pièces de serrage (64, 68) sont fixées respectivement aux premier et second ensembles (61, 65).

13. Procédé de fabrication d'un contenant de seringue avec un ensemble moule selon la revendication 12, **caractérisé en ce que** le matériau polymère est injecté dans la cavité de moule (621, 631, 661, 671) à travers une partie de la cavité de moule qui reçoit l'extrémité de la canule (51) et/ou une extrémité du noyau (69) formée avec l'orifice (691).

14. Procédé de fabrication d'un contenant de seringue avec un ensemble moule selon la revendication 13, **caractérisé en ce que** le matériau polymère est injecté dans la cavité de moule (621, 631, 661, 671) à travers un premier point d'injection (635) et un second point d'injection (675) qui s'ouvrent dans la partie de la cavité de moule (621, 631, 661, 671) et qui sont symétriques autour d'un axe longitudinal (692) du noyau (69).

15. Procédé de fabrication d'un contenant de seringue avec un ensemble moule selon la revendication 14, **caractérisé en ce que** le premier point d'injection (635) et le second point d'injection (675) sont formés respectivement dans le premier ensemble (61) et le second ensemble (65).

16. Procédé de fabrication d'un contenant de seringue avec un ensemble moule selon la revendication 15, **caractérisé en ce que** le premier ensemble (61) comporte en outre un premier bloc principal (62) et un premier sous-bloc (63) ; le second ensemble (65) comporte en outre un second bloc principal (66) et un second sous-bloc (67) ; la partie de la cavité de moule (621, 631, 661, 671) est formée entre le premier sous-bloc (63) et le second sous-bloc (67) ; une autre partie de la cavité de moule (621, 631, 661, 671) qui ne reçoit pas la canule (51) est formée entre le premier bloc principal (62) et le second bloc principal (66).

17. Procédé de fabrication d'un contenant de seringue avec un ensemble moule selon la revendication 16, **caractérisé en ce que** le premier ensemble (61) comporte deux des premiers sous-blocs (63) ; le second ensemble (65) comporte deux des seconds sous-blocs (67) ; le premier point d'injection (635) est formé entre les premiers sous-blocs (63) ; le second point d'injection (675) est formé entre les seconds sous-blocs (67).

18. Procédé de fabrication d'un contenant de seringue avec un ensemble moule selon l'une quelconque des revendications 11 à 17, **caractérisé en ce que** la première pièce de serrage (64) et la seconde pièce de serrage (68) sont fabriquées en une matière plastique.

19. Procédé de fabrication d'un contenant de seringue avec un ensemble moule selon la revendication 18, **caractérisé en ce que** la première pièce de serrage (64) est formée avec une première fente (641) dans laquelle une partie de la canule (51) vient en butée contre la première pièce de serrage (64).

20. Procédé de fabrication d'un contenant de seringue avec un ensemble moule selon la revendication 19, **caractérisé en ce que** le premier ensemble (61) est formé avec un premier conduit (633) qui fait communiquer la première fente (641) avec la cavité de moule (621, 631, 661, 671).

21. Procédé de fabrication d'un contenant de seringue avec un ensemble moule selon la revendication 20, **caractérisé en ce qu'**une autre extrémité de la canule (51) est à l'extérieur de l'ensemble moule (6) et une partie médiane (513) de la canule (51) est reçue dans le premier conduit (633).
